# EUROPÄISCHE PATENTSCHRIFT

(11) **EP 2 464 405 B1**
(45) Veröffentlichungstag und Bekanntmachung des Hinweises auf die Patenterteilung: **30.08.2017**
(21) Anmeldenummer: 10721139.3
(22) Anmeldetag: 04.05.2010
(51) Int. Cl.: A61B 1/005, A61M 25/01, G02B 23/24, A61B 17/00, A61M 25/00

(54) **MEDIZINISCHES KATHETERINSTRUMENT**
MEDICAL CATHETER INSTRUMENT
INSTRUMENT MÉDICAL À CATHÉTER

(30) Priorität: 10.08.2009 DE 102009037827
(43) Veröffentlichungstag der Anmeldung: 20.06.2012
(73) Patentinhaber: EPflex Feinwerktechnik GmbH, 72581 Dettingen/Erms (DE)
(72) Erfinder: UIHLEIN, Bernhard, 72581 Dettingen (DE)
(74) Vertreter: Patentanwälte Ruff, Wilhelm, Beier, Dauster & Partner mbB
(86) Internationale Anmeldenummer: PCT/EP2010/056039
(87) Internationale Veröffentlichungsnummer: WO 2011/018249

(56) Entgegenhaltungen:
- EP-A1- 1 484 003
- EP-A1- 1 690 564
- US-A1- 2002 161 353
- US-A1- 2002 177 766
- US-B1- 6 544 215

## Beschreibung

Die Erfindung bezieht sich auf medizinisches Katheterinstrument mit einem Schaftabschnitt, einem gegenüber dem Schaftabschnitt in wenigstens einer Richtung gesteuert biegbaren distalen Abschnitt und wenigstens einem sich axial beweglich durch den Schaftabschnitt hindurch zum distalen Abschnitt erstreckenden Steuerelement, dessen Axialbewegung eine korrespondierende Biegebewegung des distalen Abschnitts steuert.

Medizinische Katheterinstrumente dieser Art sind verschiedentlich bekannt. So offenbart die Patentschrift US 7.033.345 B2 ein Katheterinstrument, bei dem das Steuerelement von einem Zugdraht gebildet ist, der sich von einem Griffbereich des Katheters durch den Schaftabschnitt und den distalen Abschnitt hindurch innerhalb eines zugehörigen Zugdrahtlumens bis zum Vorderende des distalen Abschnitts erstreckt und dort fixiert ist. Ein im Wesentlichen mittiger Hohlkanal dient als Nutzkanal. In einem weiteren Lumen ist in den distalen Abschnitt ein im Querschnitt rechteckförmiges Gummielement eingebracht, das als Versteifungselement fungiert, welches die Rückstellung des distalen Abschnitts von seiner gebogenen Stellung in seine geradlinige Ausgangsstellung bewirken soll, wenn diese rückstellende Bewegung durch Nachlassen des Zugdrahts freigegeben wird.

Die Patentschrift EP 0 489 937 B1 offenbart ein medizinisches Katheterinstrument, bei dem als Steuerelement zur Steuerung einer aktiven Biegebewegung des distalen Abschnitts in einer Biegebewegungsebene zwei Zugseilstücke dienen, die bezüglich eines längsmittigen Nutz-Hohlkanals des Katheters gegenüberliegend angeordnet sind. Dabei verlaufen die Zugdrähte innerhalb des distalen Abschnitts direkt in einem jeweiligen Vollmaterial-Lumen, während sie innerhalb des Schaftabschnitts jeweils in einer biegesteifigkeitserhöhenden Hülse verlaufen, die ihrerseits in ein entsprechendes Vollmaterial-Lumen eingebracht ist. In der zur Biegebewegungsebene senkrechten Ebene sind in den distalen Abschnitt Bänder mit rechteckförmigem Querschnitt eingearbeitet, die den distalen Abschnitt stabilisieren und Auslenkungen desselben quer zur Biegebewegungsebene entgegenwirken sollen. Die Zugseilstücke sind als Abschnitte eines gemeinsamen, durchgehenden Zugseiles realisiert, deren Verbindungsstück an einer vorderen Endkappe des distalen Abschnitts fixiert ist.

Bei einem in der Patentschrift DE 103 37 580 B4 offenbarten Katheter ist ein Steuerelement vorgesehen, das dafür ausgelegt ist, sowohl Zugkräfte als auch Schubkräfte zu übertragen, um eine Abbiegbarkeit des distalen Katheterabschnitts in zwei entgegengesetzten Richtungen unter Verwendung nur dieses einen Steuerelements bewirken zu können.

Weitere medizinische Katheterinstrumente der eingangs erwähnten Art sind in den Offenlegungsschriften WO 97/01369 A1 und WO 95/31243 A1 offenbart.

In der Offenlegungsschrift US 2002/0161353 A1 ist ein steuerbarer Katheter mit einem verstärkten distalen Endbereich offenbart, durch den sich das distale Katheterende bevorzugt in einer gewünschten Biegeebene biegen soll und Abbiegungen senkrecht zu dieser Ebene erschwert werden sollen. Ein oder mehrere Zugdrähte erstrecken sich außermittig in einem jeweiligen Hohlkanal eines flexiblen röhrenförmigen Katheterkörpers und sind mit ihrem proximalen Ende an einem Steuergriff verankert und mit einem distalen Ende an einem distalen Endabschnitt des Katheterkörpers befestigt. Ein oder mehrere Versteifungselemente erstrecken sich längs wenigstens eines Teils der Länge des distalen Endabschnitts und sind im allgemeinen symmetrisch zu einem Durchmesser des distalen Endabschnitts bezüglich der Biegeebene desselben angeordnet, wobei sie ein Material mit einem höheren Elastizitätsmodul aufweisen als ein Kunststoffmaterial des distalen Endabschnitts. Ein ähnlicher steuerbarer Katheter ist in der Offenlegungsschrift EP 1 690 564 A1 offenbart.

Weitere herkömmliche, gesteuert biegbare medizinische Katheter- oder Führungsdrahtinstrumente sind in den Offenlegungsschriften US 2002/0177766 A1 und EP 1 484 003 A1 und der Patentschrift US 6,544,215 B1 offenbart.

Der Erfindung liegt als technisches Problem die Bereitstellung eines medizinischen Katheterinstruments der eingangs genannten Art zugrunde, das eine Abbiegbarkeit des distalen Abschnitts mit hoher Funktionszuverlässigkeit, großer Nutzungsflexibilität und/oder vergleichsweise geringem Fertigungsaufwand ermöglicht.

Die Erfindung löst dieses Problem durch die Bereitstellung eines medizinischen Katheterinstruments mit den Merkmalen des Anspruchs 1.

Dieses Katheterinstrument beinhaltet eine oder mehrere Biegeanordnungen, die jeweils wenigstens ein langgestrecktes, elastisch biegbares Versteifungselement und wenigstens ein langgestrecktes, biegbares und axial längenveränderbares Biegeelement umfassen. Das Versteifungselement und das Biegeelement sind beide wenigstens in einem axialen Teilbereich des distalen Abschnitts vorgesehen und erstrecken sich in diesem mit axialer Hauptkomponente, d.h. vollständig oder jedenfalls überwiegend in axialer Richtung. Dabei bildet das Versteifungselement ein biegebewegungsrückstellendes Element mit höherer Biegesteifigkeit als das Biegeelement. Das Biegeelement ist an das Steuerelement angekoppelt, dessen Axialbewegung die korrespondierende Biegebewegung des distalen Abschnitts steuert, oder bildet einen Teil des Steuerelements.

Das Steuerelement ist durch einen Zugdraht gebildet, der durch Hohlkanäle des Biegeelements durchgeführt ist und mit einem distalen Endbereich des Biegeelements gekoppelt ist. Das Biegeelement weist zwei Hohlkanäle auf, durch die der Zugdraht einteilig unter Bildung eines Umkehrbogens am distalen Endbereich des Biegeelements durchgeführt ist. Auf diese konstruktiv einfache Weise kann die Biegebewegung durch synchrones Ziehen an beiden proximalen Zugdrahtteilen bewirkt werden, wobei der Umkehrbogen die Zugkraft auf das Biegeelement überträgt. Als Zugdraht sind vorliegend alle für diesen Zweck herkömmlicherweise verwendeten Drahttypen geeignet, wobei der Begriff Zugdraht vorliegend der Einfachkeit halber auch Zugseile jedweder Art umfassen soll.

Das Versteifungselement und das Biegeelement können jeweils als eigenständiges Element bzw. Bauteil in den betreffenden axialen Teilbereich des distalen Abschnitts eingebracht oder integral als Teil eines Vollmaterials des distalen Abschnitts gebildet sein und in optimal anpassbarer Weise auf ihre Funktionen der rückstellenden Versteifung bzw. der aktiven Bewirkung der Biegebewegung ausgelegt werden, ohne dass gegebenenfalls anderweitiges Material des Katheterinstruments in diesem Bereich, typischerweise ein mit einem oder mehreren Lumen versehenes Vollmaterial oder eine Koaxialschlaucheinheit, auf die Erfüllung dieser Funktionen ausgelegt werden braucht. So erlaubt dies bei Bedarf beispielsweise die Verwendung eines sehr weichen Vollmaterials für den Grundaufbau des distalen Katheterabschnitts, das weder versteifend, noch biegebestimmend wirken muss. Die rückstellende Steifigkeit wird vielmehr durch das Versteifungselement gewährleistet, das z.B. als separates Element in das weiche Vollmaterial eingebracht oder von einer biegesteiferen Vollmaterialzone gebildet ist. Das Biegeelement bestimmt zusammen mit dem Steuerelement die aktive Biegebewegung. Das Biegeelement kann im Zusammenwirken mit dem Versteifungselement eine verbesserte Funktionalität hinsichtlich der aktiven Biegebewegung beispielsweise im Vergleich zu herkömmlichen Biegeanordnungen ermöglichen, bei denen ein Zugdraht-Steuerelement lediglich auf einen distalen Endkappenbereich eines homogenen Vollmaterials des distalen Abschnitts wirkt.

In Weiterbildung der Erfindung setzt sich das Versteifungselement vom distalen Abschnitt in den Schaftabschnitt hinein fort. Auf diese Weise kann es im Schaftabschnitt zur Bereitstellung der für diesen typischerweise erforderlichen, deutlich höheren Biegesteifigkeit im Vergleich zum aktiv biegbaren distalen Abschnitt beitragen.

In Weiterbildung der Erfindung weisen eine oder mehrere Komponenten des distalen Abschnitts eine Beschichtung mit einem röntgensichtbaren Material oder eine unter Magnetresonanz sichtbare Beschichtung oder Dotierung auf. Dadurch kann bei Bedarf die Röntgensichtbarkeit bzw. Magnetresonanzsichtbarkeit des Katheterinstruments verbessert werden.

In Weiterbildung der Erfindung ist das Biegeelement durch ein elastisch druckbelastbares Schraubenfeder- oder Hohlstabelement oder eine elastisch druckbelastbare Vollmaterialzone des distalen Abschnitts realisiert. Speziell kann das Biegeelement z.B. durch eine Schraubenfeder mit im unbelasteten Zustand voneinander axial beabstandeten Windungen oder eine relativ biegeweiche Vollmaterialzone des distalen Abschnitts gebildet sein.

In Weiterbildung der Erfindung ist das Versteifungselement durch ein elastisch zugbelastbares Schraubenfeder- oder Stabelement oder eine elastisch zugbelastbare Vollmaterialzone des distalen Abschnitts gebildet. Speziell kann das Versteifungselement z.B. durch eine Schraubenfeder mit im unbelasteten Zustand aneinanderliegenden Windungen oder eine relativ biegesteife Vollmaterialzone des distalen Abschnitts gebildet sein.

In Weiterbildung der Erfindung sind das Versteifungselement und das Biegeelement einander bezüglich einer Längsmittenachse des distalen Abschnitts gegenüberliegend angeordnet. Dies erlaubt mindestens eine Biegebewegung in der Ebene des Versteifungselements und des Biegeelements in der einen und/oder der anderen Biegerichtung je nachdem, ob das Biegeelement durch das Steuerelement auf Zug oder Schub belastet wird.

In Weiterbildung der Erfindung beinhaltet die Biegeanordnung wenigstens zwei Biegeelemente, die einander in der Biegebewegungsebene gegenüberliegend angeordnet sind, und wenigstens zwei Versteifungselemente, die nebeneinander in einer Ebene quer zur Biegebewegungsebene angeordnet sind. Dies ermöglicht ein Biegen des distalen Abschnitts in beiden Richtungen in der Biegebewegungsebene auch bei ausschließlicher Verwendung von nur mit Zug- und nicht mit Schubkräften arbeitenden Steuerelementen.

In Weiterbildung der Erfindung ist der distale Katheterabschnitt aus einem Vollmaterial oder aus zwei koaxialen Schlauchelementen aufgebaut, wobei die Biegeanordnung im Vollmaterial oder im Ringraum zwischen den koaxialen Schlauchelementen vorgesehen ist. Wenn das Biegeelement und das Versteifungselement als eigenständige Elemente ausgebildet sind, welche die Versteifungsfunktion bzw. die Biegeaktorfunktion erfüllen, brauchen das Vollmaterial bzw. die koaxialen Schlauchelemente nicht auf die Erfüllung dieser Funktionen ausgelegt werden. Sie brauchen lediglich so gewählt werden, dass sie diese Funktionen des Biegeelements und des Versteifungselements nicht behindern. Alternativ können das Biegeelement und/oder das Versteifungselement von einer entsprechenden Zone des Vollmaterials gebildet sein, das in diesem Fall mehrzonig aus verschiedenen Materialien aufgebaut ist.

In Weiterbildung der Erfindung weist der distale Katheterabschnitt einen mittigen axialen Nutzkanal und/oder einen oder mehrere außermittige axiale Nutzkanäle auf. Der oder die Nutzkanäle können z.B. für an sich bekannte Katheterfunktionen genutzt werden, wie zur Durchleitung eines Fluids oder Gases oder zum Betrieb eines am distalen Ende benötigten Funktionselements wie eines Spiegels, eines Messers, einer Schere, eines Sensors etc. Dazu kann sich der jeweilige Nutzkanal vorzugsweise durchgehend auch durch den Schaftabschnitt hindurch erstrecken.

In Ausgestaltung dieses Aspekts ist der distale Abschnitt aus einem den mittigen axialen Nutzkanal mit ungleichförmiger Dicke umgebenden Vollmaterial aufgebaut, wobei als Versteifungselement eine entsprechend biegesteife Vollmaterialzone fungiert, die sich in einem Bereich geringerer Dicke des Vollmaterials befindet. Dazu kann das schlauchförmige Vollmaterial z.B. in fertigungstechnisch einfacher Weise durch einen Extrusionsprozess unter Verwendung wenigstens zweier Materialien unterschiedlicher Biegesteifigkeit gebildet sein. In weiterer Ausgestaltung beinhaltet das Vollmaterial zudem eine als Biegeelement fungierende Zone in einem Bereich größerer Dicke. Auch für diese Ausgestaltung kann das Vollmaterial z.B. als entsprechend extrudiertes Schlauchbauteil gefertigt sein.

In Weiterbildung der Erfindung beinhaltet das Katheterinstrument mehrere Biegeanordnungen mit jeweils wenigstens einem Versteifungselement und wenigstens einem Biegeelement der erwähnten Art, wobei die Biegeanordnungen in verschiedenen axialen Teilbereichen des distalen Abschnitts vorgesehen sind und wenigstens zwei Biegeanordnungen zueinander nichtparallele Biegebewegungsebenen aufweisen. Dies ermöglicht ein Biegen des distalen Abschnitts in mehreren nichtparallelen Biegebewegungsebenen.

In Weiterbildung der Erfindung beinhaltet der distale Katheterabschnitt wenigstens ein Lichtleiterelement und einen daran angekoppelten Leuchtring. Dabei ist das Lichtleiterelement axial verlaufend in einem Nutzkanal des distalen Abschnitts aufgenommen. Der Leuchtring kann hierbei als Lichtquelle am distalen Vorderende des Katheters dienen.

In Weiterbildung der Erfindung weist der distale Katheterabschnitt ein axial elastisch bewegliches distales Endstück auf, das mit wenigstens einem Führungsstift verbunden ist, der in einem axialen Hohlkanal des distalen Abschnitts axial elastisch beweglich geführt ist. Auf diese konstruktiv einfache Weise ist der distale Katheterabschnitt an seinem distalen Ende axial elastisch nachgiebig, was beispielsweise Verletzungsgefahren beim Einführen des Katheterinstruments in ein Körpergewebe vorbeugen kann.

Vorteilhafte Ausführungsformen der Erfindung sind in den Zeichnungen dargestellt und werden nachfolgend beschrieben. Hierbei zeigen:
- Fig. 1: eine schematische Seitenansicht eines hier interessierenden Teils eines medizinischen Katheterinstruments mit biegbarem distalem Abschnitt,
- Fig. 2: eine schematische Perspektivansicht eines axialen Teilbereichs des biegbaren distalen Abschnitts eines Katheters nach Art von Fig. 1 mit einer Biegeanordnung mit Biegeelement und Versteifungselement,
- Fig. 3: eine Perspektivansicht des Biegeelements von Fig. 2,
- Fig. 4: eine Perspektivansicht eines als Biegebewegungs-Steuerelement für den Katheter der Fig. 1 und 2 verwendbaren Zugdrahtes,
- Fig. 5: die Ansicht von Fig. 2 mit dem Zugdraht von Fig. 4 im Einbauzustand,
- Fig. 6: die Ansicht von Fig. 5 mit aktiv gebogenem distalem Abschnitt,
- Fig. 7: eine Ansicht entsprechend Fig. 2 für eine Variante mit Schraubenfedern als Biege- und Versteifungselemente,
- Fig. 8: eine Ansicht entsprechend Fig. 2 für eine Variante mit einer Biegeanordnung mit je einem Paar gegenüberliegender Biegeund Versteifungselemente,
- Fig. 9: eine Querschnittansicht durch den distalen Abschnitt längs einer Linie IX-IX von Fig. 1 für die Variante von Fig. 8,
- Fig. 10: eine Querschnittansicht durch den Schaftabschnitt, längs einer Linie X-X der Kathetervariante von Fig. 8,
- Fig. 11: eine Ansicht entsprechend Fig. 10 für eine Variante mit zusätzlichen Versteifungselementen in Schaftabschnitt,
- Fig. 12: eine Ansicht entsprechend Fig. 9 für eine Variante mit zusätzlichen außermittigen Nutzkanälen,
- Fig. 13: eine Ansicht entsprechend Fig. 10 für die Variante von Fig. 12,
- Fig. 14: eine Ansicht entsprechend Fig. 11 für die Variante von Fig. 12,
- Fig. 15: eine Ansicht entsprechend Fig. 12 für eine Schlauchvariante,
- Fig. 16: eine Perspektivansicht des distalen Abschnitts einer Kathetervariante mit Biegbarkeit in zwei nichtparallelen Ebenen,
- Fig. 17: eine Ansicht entsprechend Fig. 16, wobei zusätzlich ein an den distalen Abschnitt angrenzender Teil des Schaftabschnitts dargestellt ist,
- Fig. 18: eine schematische Perspektivansicht eines vorderen Endbereichs einer Kathetervariante mit distalem Leuchtelement,
- Fig. 19: eine Längsschnittansicht des distalen Endbereichs einer Kathetervariante mit axial elastisch gehaltenem Endstück,
- Fig. 20: eine Ansicht entsprechend Fig. 9 für eine Variante mit Vollmaterialzone als Biegeelement,
- Fig. 21: eine Ansicht entsprechend Fig. 10 für die Variante von Fig. 20,
- Fig. 22: eine Ansicht entsprechend Fig. 20 für eine Variante mit einer Vollmaterialzone als Versteifungselement,
- Fig. 23: eine Ansicht entsprechend Fig. 22 für eine modifizierte Versteifungselement-Vollmaterialzone,
- Fig. 24: eine Ansicht entsprechend Fig. 23 für eine Variante mit versetztem mittigem Nutzkanal,
- Fig. 25: eine Ansicht entsprechend Fig. 21 für die Variante von Fig. 24,
- Fig. 26: eine Ansicht entsprechend Fig. 24 für eine Ausführungsform der Erfindung mit zurückgeschleiftem Zugdraht,
- Fig. 27: eine Längsschnittansicht längs einer Linie A-A von Fig. 26 und
- Fig. 28: eine Ansicht entsprechend Fig. 12 für eine Ausführungsform der Erfindung mit zurückgeschleiften Zugdrähten.

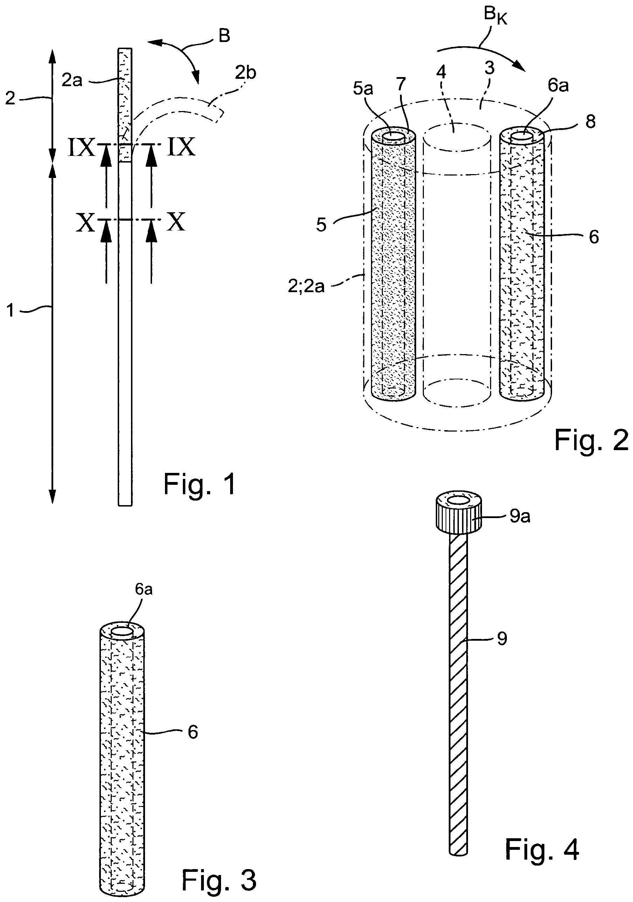

In Fig. 1 ist lediglich schematisch ein Schlauchteil eines medizinischen Katheterinstruments dargestellt. Der Schlauchteil beinhaltet einen Schaftabschnitt 1 und einen biegbaren Abschnitt 2. Ein sich proximal an den Schaftabschnitt 1 in einer üblichen Weise anschließender Griffteil des Instruments ist der Einfachkeit halber weggelassen. Der distale Abschnitt 2 ist zwischen einer im gezeigten Beispiel geradlinigen ersten Endstellung 2a und einer gestrichelt angedeuteten, maximal gebogenen zweiten Endstellung 2b gesteuert biegbar, wie durch einen Biegepfeil B symbolisiert.

Fig. 2 zeigt in einer schematischen Perspektivansicht den distalen Abschnitt 2 in seiner geradlinigen Endstellung 2a für eine konkrete, vorteilhafte Ausführungsform. Wie daraus ersichtlich, sind in ein Schlauch-Vollmaterial 3 ein längsmittiges Lumen 4, das als ein axialer Nutzkanal des Katheters fungiert, sowie ebenfalls axial verlaufend ein Versteifungselement 5 und ein Biegeelement 6 eingebracht.

Das Biegeelement 6 ist als separates Bauteil in Fig. 3 gezeigt und weist eine Hohlstabform mit einem mittigen Hohlkanal 6a auf. In gleicher Weise ist das Versteifungselement 5 als stabförmiges Element mit einem längsmittigen Hohlkanal 5a gebildet. Das Biegeelement 6 ist aus einem elastischen Material derart gebildet, dass es elastisch biegbar und dabei insbesondere auch in seiner axialen Ausdehnung elastisch längenveränderlich, insbesondere kontrahierbar, ist. Das Versteifungselement 5 weist eine gegenüber dem Biegeelement 6 und dem Vollmaterial 3 höhere Biegesteifigkeit auf. Das Versteifungselement 5 und das Biegeelement 6 sind jeweils in einen entsprechenden axialen Hohlkanal 7, 8 des Vollmaterials 3 derart eingebracht, dass sie sich bezüglich der Längsmittenachse des distalen Katheterabschnitts 2 gegenüberliegen.

Das Versteifungselement 5 und das Biegeelement 6 können z.B. durch entsprechende Schlauchstücke mit unterschiedlichen elastischen Materialien realisiert sein, wobei das Schlauchmaterial für den Biegeschlauch 6 deutlich weicher als dasjenige für den Versteifungsschlauch 5 ist, so dass das Biegeelement 6 nicht nur biegefähig, sondern auch axial im benötigten Maß kontrahierbar ist, während das Versteifungselement 5 ein druckstabileres Element ist, das nicht oder jedenfalls weniger axial komprimierbar ist. Das Katheterschlauch-Vollmaterial 3 kann noch weicher als dasjenige des Biegeelements 6 sein und jedenfalls im distalen Abschnitt 2 einen Multilumenschlauch mit den wenigstens drei axialen Hohlkanälen 4, 7, 8 bilden, wie in Fig. 2 gezeigt.

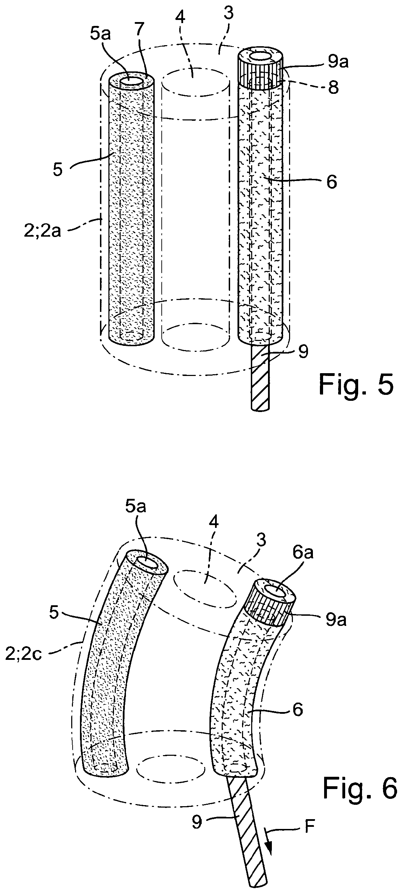

Die mit dem Versteifungselement 5 und dem Biegeelement 6 realisierte Biegeanordnung ermöglicht ein aktives Biegen des distalen Abschnitts aus seiner geradlinigen Ausgangsstellung gemäß Fig. 2 in eine gebogene Stellung, wie mit einem Biegepfeil B_{K} symbolisiert. Um ein gesteuertes Biegen des distalen Abschnitts zu bewirken, ist ein Steuerelement in Form eines in Fig. 4 gezeigten Zugdrahtes 9 vorgesehen, der an einem Ende mit einem verdickten Kopfteil 9a versehen ist. Im montierten, in Fig. 5 gezeigten Zustand erstreckt sich der Zugdraht 9 durch den Hohlkanal 6a des Biegeelements 6 hindurch, wobei er sich mit seinem Kopfteil 9a distal über das Biegeelement 6 hinaus erstreckt und gegen dieses axial in Zugrichtung anliegt. Es versteht sich, dass alternativ zu dieser Kopfteilgestaltung die gewünschte, zugbelastbare Ankopplung des distalen Zugdrahtendes an das distale Biegeelementende auch in anderer, herkömmlicher Weise realisierbar ist, z.B. durch Kleben, Schweißen oder Löten. In herkömmlicher und daher nicht weiter gezeigter Weise erstreckt sich der Zugdraht 9 in proximaler Richtung durch den Schaftabschnitt 1 hindurch zu einem Griff-/Betätigungsteil des Katheterinstruments. Dort kann er von einem Benutzer auf Zug belastet werden, wie in Fig. 6 durch einen zugehörigen Zugkraftpfeil F symbolisiert. Die Zugbelastung wird vom Zugdraht 9 über dessen Kopfteil 9a auf das Biegeelement 6 übertragen.

Da das Biegeelement 6 axial elastisch kontrahierbar ist und das Versteifungselement 5 eine gegenüber dem Biegeelement 6 höhere Biegesteifigkeit aufweist, hat die Zugkrafteinwirkung auf das Biegeelement 6 zur Folge, dass sich dieses axial verkürzt, während sich die axiale Länge des Versteifungselements 5 nicht oder jedenfalls nur weniger als diejenige des Biegeelements 6 verringert oder sich sogar erhöht. Dies bewirkt insgesamt ein abbiegendes Krümmen des Biegeelements 6, des biegeelastischen Versteifungselements 5 und des biegsamen Vollmaterials 3 in eine gekrümmte Stellung 2c in Richtung auf die Seite des Biegeelements 6, wie in Fig. 6 dargestellt. Dabei ist die Biegestellung zwischen den beiden Endstellungen 2a, 2b von Fig. 1 durch entsprechende Betätigung des Zugdrahtes 9 variabel einstellbar, z.B. stufenlos.

Wenn die Zugkraftbelastung am Zugdraht 9 wieder gelöst wird, drückt das Versteifungselement 5 dank seiner Biegesteifigkeit den distalen Katheterabschnitt 2 wieder in seine Ausgangsstellung zurück, im gezeigten Beispiel die geradlinige Ausgangsstellung 2a gemäß Fig. 5, welche gleichzeitig die unbelastete Ausgangsstellung des Versteifungselements 5 darstellt, wobei in alternativen Gestaltungen die unbelastete Ausgangsstellung auch eine gekrümmte Stellung des distalen Abschnitts 2 sein kann.

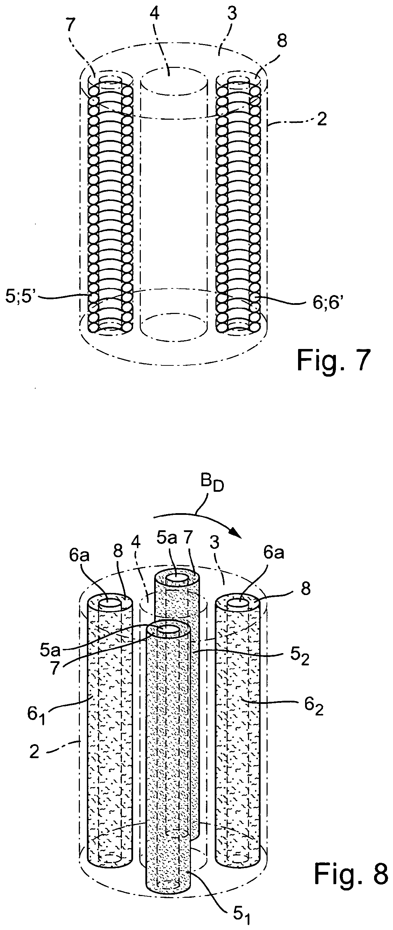

Fig. 7 zeigt eine Kathetervariante, bei der für die Biegeanordnung das Versteifungselement 5 und das Biegeelement 6 durch je eine Schraubenfeder 5', 6' realisiert sind. Dabei grenzen bei der Versteifungselement-Schraubenfeder 5' deren Wicklungen im unbelasteten Ausgangszustand mit Berührkontakt aneinander an, d.h. die Schraubenfeder 5' und entsprechend das Versteifungselement 5 ist axial nicht kontrahierbar und daher druckstabil, jedoch elastisch zugbelastbar und somit biege- und dehnungsfähig. Hingegen sind bei der Biegeelement-Schraubenfeder 6' die benachbarten Wicklungen axial beabstandet, so dass das dadurch gebildete Biegeelement 6 nicht nur biege- und dehnfähig, sondern auch elastisch druckbelastbar und daher axial kontrahierbar ist, wie es für die auf Zug arbeitende Biegeelementfunktion gefordert ist. Dabei sind in Fig. 7 und in allen weiteren Figuren zum leichteren Verständnis übereinstimmende Bezugszeichen nicht nur für identische, sondern auch für funktionell äquivalente Komponenten verwendet. Das Innere der jeweiligen Schraubenfeder 5', 6' bildet den entsprechenden Hohlkanal 5a, 6a des Versteifungselements 5' bzw. des Biegeelements 6'.

Fig. 8 zeigt eine Kathetervariante, bei welcher der distale Abschnitt 2 ausgehend von der gezeigten geradlinigen, unbelasteten Ausgangsstellung in beide entgegengesetzte Richtungen einer zugehörigen Biegeebene biegbar ist, wie durch einen Biegepfeil B_{D} symbolisiert. Dazu weist die Biegeanordnung des distalen Abschnitts 2 in diesem Fall zwei Biegeelemente 6₁, 6₂ und zwei Versteifungselemente 5₁, 5₂ auf. Die beiden Biegeelemente 6₁, 6₂ sind jeweils axial verlaufend und einander bezüglich der Längsmittenachse des distalen Katheterabschnitts 2 gegenüberliegend in entsprechende Hohlkanäle des Vollmaterials 3 eingebracht. Analog sind die beiden Versteifungselemente 5₁, 5₂ axial verlaufend und sich bezüglich der Längsmittenachse des distalen Katheterabschnitts 2 gegenüberliegend in entsprechende Hohlkanäle des Vollmaterials 3 eingebracht, wobei sie in Umfangsrichtung des Vollmaterials 3 um 90° versetzt zu den Biegeelementen 6₁, 6₂ angeordnet sind. Die Biegeelemente 6₁, 6₂ und die Versteifungselemente 5₁, 5₂ können von irgendeiner der oben zu den Fig. 1 bis 7 erwähnten Bauarten sein.

Jedem der beiden Biegeelemente 6₁, 6₂ ist jeweils ein Steuerelement nach Art des Zugdrahts 9 von Fig. 4 zugeordnet. Wenn der Zugdraht, der dem in Fig. 8 rechten Biegeelement 6₂ zugeordnet ist, zugbelastet wird, drückt er auf dieses Biegeelement 6₂, das sich dadurch verkürzt.

Zusammen mit den druckstabilen Versteifungselementen 5₁, 5₂ bewirkt dies ein Abbiegen des distalen Abschnitts 2 zur Seite des druckbelasteten Biegeelements 6₂ hin, d.h. in Fig. 8 nach rechts, wobei natürlich keine Zugkraft auf das andere Biegeelement 6₁ ausgeübt wird. Wird umgekehrt das in Fig. 8 linke Biegeelement 6₁ druckbelastet und bleibt das in Fig. 8 rechte Biegeelement 6₂ entlastet, biegt sich der distale Abschnitt 2 aus seiner geradlinigen Stellung von Fig. 8 zu dieser Seite hin, d.h. in Fig. 8 nach links.

Die Ebene, in welcher die beiden Biegeelemente 6₁, 6₂ liegen, bildet folglich eine Biegeebene, in welcher der distale Katheterabschnitt 2 aus seiner geradlinigen Ausgangsstellung heraus wahlweise in der einen oder der anderen seiner beiden entgegengesetzten Biegerichtungen abbiegbar ist. Die beiden Versteifungselemente 5₁, 5₂ liegen in einer zur Biegeebene nichtparallelen, im gezeigten Beispiel speziell senkrechten Längsebene des distalen Katheterabschnitts 2 und fungieren als biegesteifigkeitsbestimmende Elemente, welche jeweils die Rückstellung des gebogenen distalen Katheterabschnitts 2 in seine geradlinige Ausgangsstellung bewirken, wenn die biegesteuernde Zugkraft am betreffenden Zugdraht gelöst wird.

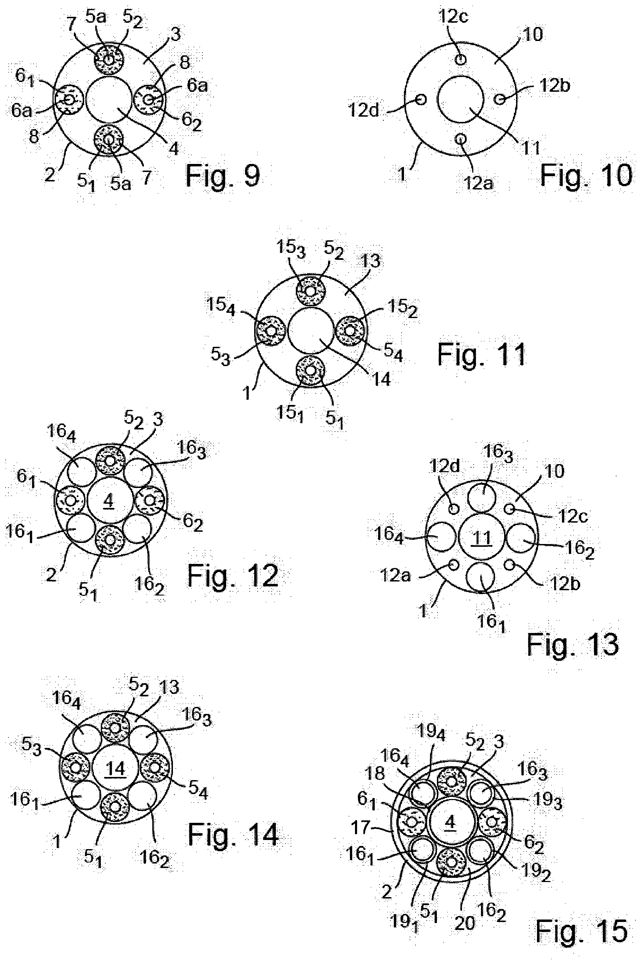

Die Fig. 9 bis 15 veranschaulichen in schematischen Querschnittansichten verschiedene Kathetervarianten hinsichtlich Gestaltung des distalen Katheterabschnitts 2 und des Schaftabschnitts 1.

Speziell zeigt Fig. 9 eine Querschnittansicht längs einer Linie IX-IX von Fig. 1 im distalen Katheterabschnitt 2 für die Kathetervariante von Fig. 8. Zu erkennen sind das den Mehrlumenschlauch bildende Vollmaterial 3, der mittige Nutzkanal 4, die beiden Versteifungselemente 5₁ und 5₂ sowie die beiden Biegeelemente 6₁ und 6₂.

Fig. 10 zeigt eine Querschnittansicht längs einer Linie X-X im Schaftabschnitt 1 von Fig. 1 für eine Schaftrealisierung aus einem Mehrlumenschlauch-Vollmaterial 10 mit einem längsmittigen Hohl-/Nutzkanal 11 und vier um 90° versetzt außermittig angeordneten Hohl-/Nutzkanälen 12a, 12b, 12c, 12d. Der mittige Nutzkanal 11 korrespondiert mit dem mittigen Nutzkanal 4 des distalen Abschnitts, d.h. bildet einen entsprechenden, durch den Schaftabschnitt 1 und den distalen Abschnitt 2 durchgehenden Nutzkanal, während jeder der außermittigen Nutzkanäle 12a bis 12d mit je einem der Hohlkanäle 5a, 6a korrespondiert, die in den beiden Versteifungselementen 5₁, 5₂ und den beiden Biegeelementen 6₁, 6₂ vorgesehen sind. Dabei dienen die den Biegeelementen 6₁, 6₂ zugeordneten Hohlkanäle 12b, 12d zur Durchführung des jeweiligen Zugdrahtes, während die beiden anderen Hohlkanäle 12a, 12c anderweitig genutzt werden können. Das Vollmaterial 10 des Schaftabschnitts 1 weist bevorzugt eine höhere Steifigkeit auf als das Vollmaterial 3 des distalen Abschnitts 2.

Fig. 11 zeigt eine Variante, bei welcher der Schaftabschnitt 1 durch ein Mehrlumenschlauch-Vollmaterial 13 gebildet ist, in den ein mittiger Hohl-/Nutzkanal 14 und vier außermittig in Umfangsrichtung äquidistant angeordnete Hohlkanäle 15₁, 15₂, 15₃, 15₄ eingebracht sind, in denen je ein Versteifungselement 5₁, 5₂, 5₃, 5₄ aufgenommen ist. Bei dem einen Paar 5₁, 5₂ sich gegenüberliegender Versteifungselemente kann es sich um diejenigen der Variante von Fig. 8 handeln, indem sich diese vom distalen Abschnitt 2 in den Schaftabschnitt 1 hinein fortsetzen und sich dabei über einen Teil oder die ganze Länge des Schaftabschnitts 1 erstrecken und zu dessen zusätzlicher Versteifung beitragen. Die beiden anderen Versteifungselemente 5₃ und 5₄ erstrecken sich nur im Schaftabschnitt 1, und an diese schließen sich im distalen Abschnitt die beiden Biegeelemente 6₁, 6₂ an. Die Hohlkanäle 7, 8, 15₁ bis 15₄ zur Aufnahme der Biegeelemente 6₁, 6₂ und Versteifungselemente 5₁ bis 5₄ können sich z.B. mit gleichem Durchmesser durchgehend durch den Schaftabschnitt 1 und den distalen Abschnitt 2 hindurch erstrecken. Das Vollmaterial 13 des Schaftabschnitts 1 kann auf Wunsch bei dieser Variante gleich dem Vollmaterial 3 des distalen Abschnitts 2 sein, wobei dann die erhöhte Biegesteifigkeit des Schaftabschnitts 1 durch die zusätzlichen Versteifungselemente 15₂, 15₄ gewährleistet wird.

Fig. 12 zeigt eine Variante, bei der ausgehend von der Variante der Fig. 8 und 9 vier zusätzliche Nutz-Hohlkanäle 16₁, 16₂, 16₃, 16₄ in das Mehrlumenschlauchmaterial 3 des distalen Abschnitts 2 eingebracht sind. Ausgehend hiervon zeigt Fig. 13 eine Variante, bei der sich diese zusätzlichen Nutz-Hohlkanäle 16₁ bis 16₄ auch durch den Schaftabschnitt 1 hindurch erstrecken, wozu das entsprechende Mehrlumenschlauch-Vollmaterial 10 von Fig. 10 mit diesen vier zusätzlichen Lumen 16₁ bis 16₄ versehen ist. Die vier zusätzlichen Nutz-Hohlkanäle 16₁ bis 16₄ können für zusätzliche Katheterfunktionen verwendet werden, z.B. zum Durchführen von Lichtfasern, Laserfasern, elektrischen Leitungen zur Signalverarbeitung oder Durchführung von Messungen oder zum Durchführen von Fluiden bzw. Gasen.

Fig. 14 zeigt eine Variante, bei welcher die vier zusätzlichen Nutz-Hohlkanäle 16₁ bis 16₄ zusätzlich zu den Hohlkanälen 15₁ bis 15₄ zur Aufnahme der Versteifungselemente 5₁ bis 5₄ in das Mehrvolumenschlauch-Vollmaterial 13 des Schaftabschnitts 1 der Variante von Fig. 11 eingebracht sind.

Fig. 15 zeigt eine funktionell der Variante von Fig. 12 entsprechende Variante, bei welcher der gezeigte distale Abschnitt 2 zwei koaxiale Schlauchelemente 17, 18 an Stelle eines Mehrlumenschlauch-Vollmaterials beinhaltet, speziell einen Außenschlauch 17 und einen Innenschlauch 18. Der Innenschlauch 18 definiert in seinem Inneren den mittigen Nutz-/Hohlkanal 4, während der Außenschlauch 17 mit dem Innenschlauch 18 einen Ringraum 20 begrenzt, in welchen die beiden Biegeelemente 6₁, 6₂ und die beiden Versteifungselemente 5₁ 5₂ sowie vier Schlauchstücke 19₁, 19₂, 19₃, 19₄ eingefügt sind, welche die vier außermittigen Nutz-Hohlkanäle 16₁ bis 16₄ definieren. Gegenüber den Mehrlumenschlauch-Vollmaterialvarianten hat diese Katheterschlauchvariante den Vorteil, dass die Schlauchmaterialien für die einzelnen Schlauchelemente bzw. Schlauchstücke variabel und beliebig in optimaler Anpassung an den Anwendungszweck ausgewählt werden können und der Fertigungsaufwand gegenüber einem vergleichbaren Mehrlumenschlauch-Vollmaterial reduziert werden kann.

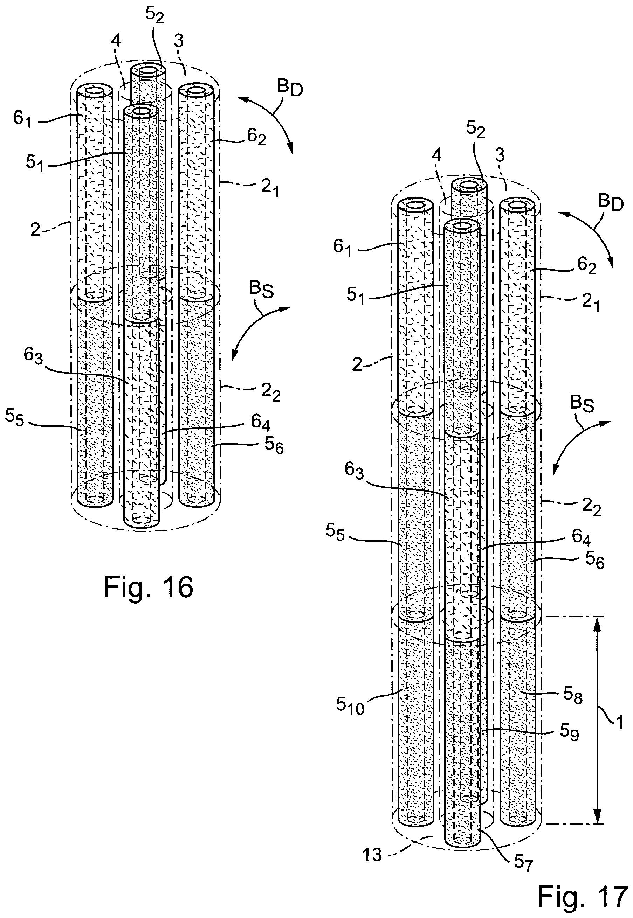

Fig. 16 zeigt eine Kathetervariante, bei welcher der biegbare distale Abschnitt 2 mehrere Biegeanordnungen in unterschiedlichen axialen Teilbereichen aufweist, speziell eine erste Biegeanordnung mit zwei gegenüberliegenden Biegeelementen 6₁, 6₂ und zwei gegenüberliegenden Versteifungselementen 5₁, 5₂ entsprechend der Biegeanordnung von Fig. 8 in einem ersten Teilbereich 2₁ und eine zweite Biegeanordnung mit zwei gegenüberliegenden Biegeelementen 6₃, 6₄ und zwei gegenüberliegenden Versteifungselementen 5₅, 5₆ ebenfalls nach Art der Biegeanordnung von Fig. 8 in einem an den ersten proximal anschließenden zweiten Teilbereich 2₂. Dabei ist die zweite Biegeanordnung gegenüber der ersten Biegeanordnung um 90° verdreht angeordnet, d.h. in axialer Richtung schließen an die beiden Versteifungselemente 5₅, 5₆ der zweiten Biegeanordnung die beiden Biegeelemente 6₁, 6₂ der ersten Biegeanordnung an, während an die beiden Biegeelemente 6₃, 6₄ der zweiten Biegeanordnung die Versteifungselemente 5₁, 5₂ der ersten Biegeanordnung anschließen.

Dies hat zur Folge, dass in der Zeichnung von Fig. 16 die Biegeebene der ersten Biegeanordnung in der Zeichenebene liegt und der distale Abschnitt in diesem Teilbereich 2₁ in beiden Richtungen in dieser Biegeebene aus der gezeigten, geradlinigen Ausgangsstellung heraus gesteuert gebogen werden kann, siehe den Biegepfeil B_{D} entsprechend Fig. 8, während sich der distale Abschnitt 2 in seinem proximalen Teilbereich 2₂ durch die dortige zweite Biegeanordnung in einer zur Zeichenebene senkrechten Biegeebene gesteuert in beide entgegengesetzte Richtungen aus seiner gezeigten, geradlinigen Ausgangsstellung heraus biegen lässt, wie durch einen Biegepfeil B_{S} symbolisiert. Zur Aufnahme der Biegeelemente 6₁ bis 6₄ und der Versteifungselemente 5₁, 5₂, 5₅, 5₆ sind entsprechend vier axiale Hohlkanäle durchgehend durch die beiden Teilbereiche 2₁, 2₂ des distalen Abschnitts 2 in das zugehörige Mehrlumenschlauch-Vollmaterial 3 eingebracht. Jedem Biegeelement 6₁ bis 6₄ jeder der beiden Biegeanordnungen ist wiederum, wie oben zur Fig. 8 erläutert, ein Steuerelement z.B. in Form eines Zugdrahtes zugeordnet, wobei die (in Fig. 16 nicht explizit gezeigten) Zugdrähte für die beiden Biegeelemente 6₁, 6₂ der vorderen Biegeanordnung durch die Hohlkanäle dieser Biegeelemente 6₁, 6₂ und durch die Hohlkanäle der proximal anschließenden Versteifungselemente 5₅, 5₆ der hinteren Biegeanordnung durchgeführt sind.

Folglich kann in dieser Kathetervariante der distale Abschnitt 2 in zwei nichtparallelen Biegeebenen, hier speziell zwei orthogonalen Biegeebenen, jeweils in entgegengesetzte Richtungen gesteuert gebogen werden, d.h. ausgehend von seiner geradlinigen Ausgangsstellung von Fig. 16 nach links und rechts sowie nach vorn und hinten. Durch Kombinieren dieser beiden nichtparallelen Biegebewegungen, hier speziell orthogonalen Biegebewegungen, lässt sich der distale Abschnitt 2 bei dieser Variante in jede gewünschte Richtung gesteuert biegen. Für die Biegeelemente 6₁ bis 6₄ und die Versteifungselemente 5₁, 5₂, 5₅, 5₆ sind wiederum alle zu den obigen Ausführungsbeispielen beschriebenen Typen verwendbar. Es versteht sich, dass bei Bedarf die beiden Biegeanordnungen alternativ auch mit axialem Abstand vorgesehen sein können und/oder der distale Abschnitt 2 eine oder mehrere weitere Biegeanordnungen in anderen axialen Teilbereichen aufweisen kann.

Fig. 17 zeigt den mit den beiden Biegeanordnungen versehenen distalen Abschnitt 2 gemäß Fig. 16 in Ankopplung an den Schaftabschnitt 1, der in diesem Fall gemäß der Variante von Fig. 11 realisiert ist, d.h. aus dem Mehrlumenschlauch-Vollmaterial 13 mit den vier Hohlkanälen 15₁ bis 15₄ zur Aufnahme je eines Versteifungselements. Dazu können in diese Hohlräume 15₁ bis 15₄ vier einzelne Versteifungselemente 5₇, 5₈, 5₉, 5₁₀ eingebracht sein, wie gezeigt. Alternativ können sich die Versteifungselemente 5₅, 5₆ der hinteren Biegeanordnung des distalen Abschnitts 2 in den Schaftabschnitt 1 hinein einteilig fortsetzen und dort die entsprechenden beiden gegenüberliegenden Versteifungselemente bilden. Das Mehrlumenschlauch-Vollmaterial 13 des Schaftabschnitts 1 kann auf Wunsch auch das Mehrlumenschlauch-Vollmaterial 3 des distalen Abschnitts sein, d.h. der gesamte Katheterschlauch aus Schaftabschnitt 1 und distalem Abschnitt 2 beinhaltet in diesem Fall ein einheitlich durchgehendes Mehrlumenschlauch-Vollmaterial. In dieses können zur Aufnahme der Biegeelemente 6₁ bis 6₄ und der Versteifungselemente 5₁, 5₂, 5₅ bis 5₁₀ vier axial durch den Schaftabschnitt 1 und den distalen Abschnitt 2 hindurchgehende Hohlkanäle jeweils um 90° in Umfangsrichtung versetzt eingebracht sein.

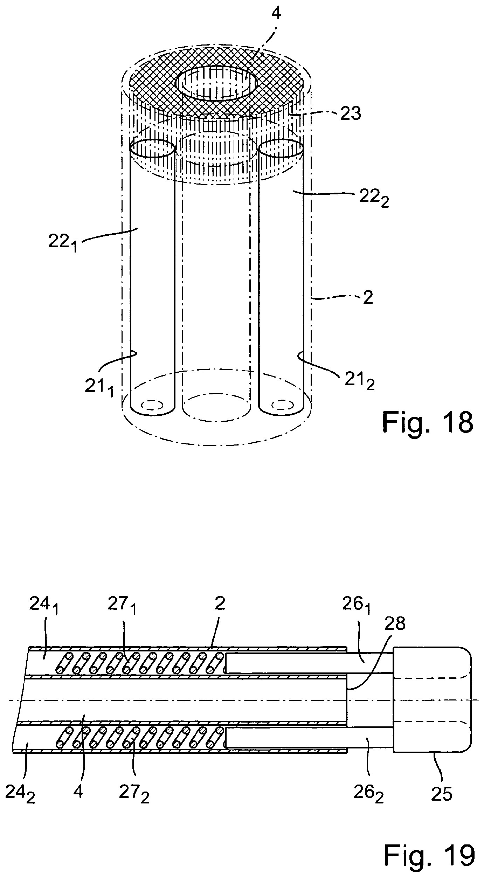

Die Fig. 18 und 19 veranschaulichen Kathetervarianten mit speziellen Nutzungsmöglichkeiten von distal endseitigen Nutz-/Hohlkanälen, wie sie im distalen Abschnitt 2 beispielsweise in Form des oder der Versteifungselement-Hohlkanäle 5a in den Varianten der Fig. 5 bis 11, 16 und 17 und/oder den zusätzlichen Nutz-/Hohlkanälen 16₁ bis 16₄ in den Varianten der Fig. 12 bis 15 vorhanden sind.

Bei der Variante von Fig. 18 werden zwei axiale Hohlkanäle 21₁, 21₂ des distalen Abschnitts 2 zur Durchführung je eines Lichtleiters 22₁, 22₂ genutzt, die distal an einem Leuchtring 23 enden, der als Beleuchtungselement am distalen Katheterende fungiert. Über die Lichtleiter 22₁, 22₂ wird der Leuchtring 23 beleuchtet, der auf diese Weise eine gleichmäßig diffuse Beleuchtung am distalen Katheterende ermöglicht.

Die Kathetervariante von Fig. 19 nutzt außermittige axiale Hohlkanäle 24₁, 24₂ zur axial elastisch nachgiebigen Lagerung eines distalen End- bzw. Kopfstücks 25 des Katheters. Dazu sind am Kopfstück 25 zwei oder mehr axiale Führungsstifte 26₁, 26₂ vorgesehen, von denen jeder in einen der zugehörigen Hohlkanäle 24₁, 24₂ hineinragt. In die Hohlkanäle 24₁, 24₂ ist je eine komprimierbare Schraubenfeder 27₁, 27₂ eingebracht, die sich in einer nicht näher gezeigten, herkömmlichen Weise an ihrem proximalen Ende geeignet abstützt. Dadurch ist das Kopfstück 25 gegenüber einem distalen Katheterschlauchende 28 axial elastisch beweglich bzw. nachgiebig angeordnet. Diese Variante ist beispielsweise für Anwendungen von Vorteil, bei denen der Katheter mit seinem distalen Ende immer dicht an einer Gefäßwand anliegen sollte, auch wenn sich diese bewegt oder nachgiebig ist.

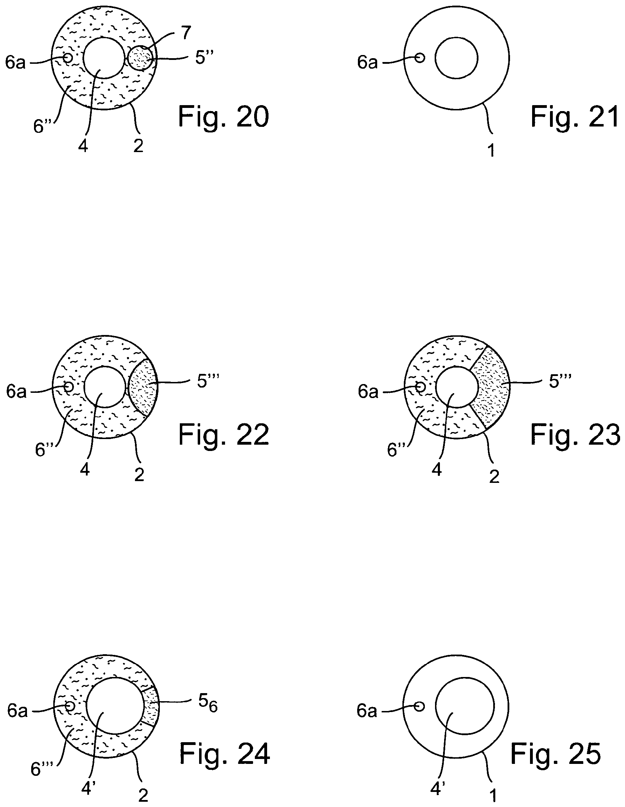

Die Figuren 20 bis 25 veranschaulichen Kathetervarianten, bei denen der distale Abschnitt von einem schlauchförmigen Vollmaterial gebildet ist, das gleichzeitig das Biegeelement bereitstellt.

In der Variante der Figuren 20 und 21 besteht dazu der schlauchförmige distale Abschnitt 2 in seinem gesamten, kreisringförmigen Querschnitt aus einem als entsprechendes Biegeelement 6" fungierenden Vollmaterial, in das der Hohlkanal 6a zur Durchführung des zugehörigen Zugdrahtes und der axiale Hohlkanal 7 eingebracht sind, in dem wie im Ausführungsbeispiel von Fig. 2 ein stabförmiges Versteifungselement 5" aufgenommen ist, das in diesem Beispiel voll massiv als separates Bauteil oder als z.B. extrudierter, integraler Teil des Vollmaterials ausgeführt ist. Der durch den Hohlkanal 6a durchgeführte Zugdraht ist mit dem distalen Ende des Vollmaterial-Biegeelements 6" auf eine der zu den obigen Beispielen beschriebene Weise gekoppelt. Der Schaftabschnitt 1 ist von einem schlauchförmigen Vollmaterial gebildet, in das lediglich der Hohlkanal (6a) zur Zugdrahtdurchführung eingebracht ist.

Das Biegeverhalten der in den Fig. 20 und 21 gezeigten Variante entspricht demjenigen der oben zu den Fig. 2 bis 6 beschriebenen Variante. Durch Ziehen am Zugdraht verkürzt sich der Vollmaterial-Biegebereich 6" in seinem in Fig. 20 linken Bereich, wodurch sich der distale Abschnitt 2 zur in Fig. 20 linken Seite hin krümmt, da das Versteifungselement 5" axial längenstabiler bleibt.

Die Fig. 22 veranschaulicht eine Variante, bei der das Biegeelement wie im Beispiel der Fig. 20 und 21 von einem den distalen Abschnitt 2 bildenden schlauchförmigen Vollmaterial 6" bereitgestellt ist, wobei in dieser Variante auch das Versteifungselement durch eine entsprechende Zone 5'" des gesamten ringförmigen Vollmaterials gebildet ist. Mit anderen Worten ist bei diesem Ausführungsbeispiel der ringförmige Vollmaterialquerschnitt des distalen Abschnitts 2 in einen das Biegeelement 6" bildenden Teilquerschnitt und einen das Versteifungselement 5'" bildenden Teilquerschnitt aufgeteilt, wobei das Biegeelement 6" den überwiegenden Querschnitt einnimmt und sich das Versteifungselement 5'" auf eine äußere Teilzone mit deutlich kleinerer Querschnittsfläche beschränkt, wie aus Fig. 22 zu erkennen. Der Schaftabschnitt entspricht bei diesem Beispiel demjenigen der Variante gemäß den Fig. 20 und 21. Bei der Kathetervariante von Fig. 22 kann der distale Abschnitt 2 insgesamt in fertigungstechnisch vorteilhafter Weise durch einen Extrusionsprozess hergestellt werden, bei dem ein biegeweicheres Material für den Biegeelement-Teilquerschnitt 6" und ein biegesteiferes Material für den Versteifungselement-Teilquerschnitt 5'" verwendet wird. Im übrigen entspricht die Kathetervariante von Fig. 22 in ihrem Biegeverhalten der Kathetervariante der Fig. 20 und 21.

Fig. 23 zeigt eine Modifikation der Kathetervariante von Fig. 22 dahingehend, dass der Versteifungselement-Teilquerschnitt 5'" ringsegmentförmig ausgebildet ist, wobei sich die Versteifungselement-Vollmaterialzone 5'" über einen Umfangswinkel von z.B. zwischen 90° und 140° erstreckt, während die Biegeelement-Vollmaterialzone 6" den übrigen, größeren Teilquerschnitt einnimmt. Wiederum entspricht die Variante von Fig. 23 in ihrem Biegeverhalten den Varianten der Fig. 20 bis 22. Je größer der Anteil der Versteifungselement-Vollmaterialzone 5'" am Gesamtquerschnitt gewählt ist, desto weniger biegesteif kann das zugehörige Material gewählt sein und/oder desto höher ist ihre Biegesteifigkeit.

Die Fig. 24 und 25 veranschaulichen eine Modifikation der Kathetervariante von Fig. 23 dahingehend, dass ein mittiger Nutzkanal 4' radial gegenüber dem Außenmantel des Katheterschlauchs versetzt vorgesehen ist, in den Fig. 24 und 25 wie gezeigt nach rechts versetzt. Dadurch umgibt das Vollmaterial des Katheterschlauchs den mittigen Nutzkanal 4' sowohl im Schaftabschnitt 1 als auch im distalen Abschnitt 2 mit unterschiedlicher Dicke, im gezeigten Fall mit auf der rechten Seite minimaler und auf der linken Seite maximaler Dicke. Im rechten Bereich geringerer Dicke ist ein ringsegmentförmiger Teil des Querschnitts des distalen Abschnitts 2 als Vollmaterial-Versteifungszone 5₆ gebildet, und das restliche Vollmaterial einschließlich des Bereichs größerer Dicke bildet ein Biegeelement 6"'.

Wie aus den Fig. 24 und 25 im Vergleich zu den Fig. 20 bis 23 ersichtlich, ermöglicht diese radiale Versetzung des mittigen Nutzkanals 4 einen größeren Querschnitt des Nutzkanals 4 und damit ein größeres Nutzvolumen bei gleichem Außendurchmesser des Katheterschlauchs. Bei Wahl eines entsprechend biegesteifen Materials genügt die Versteifungselement-Vollmaterialzone 5₆ im Bereich geringerer Vollmaterialdicke zur Bereitstellung der erforderlichen Biegesteifigkeit des distalen Abschnitts 2, wobei wie gezeigt auch bereits eine Ringsegmenterstreckung von deutlich weniger als 90° für die Versteifungselement-Vollmaterialzone 5₆ ausreichen kann. Auch im Ausführungsbeispiel der Fig. 24 und 25 kann der distale Abschnitt 2 in fertigungstechnisch einfacher Weise durch Extrudieren unter Verwendung zweier Materialien unterschiedlicher Biegesteifigkeit hergestellt werden.

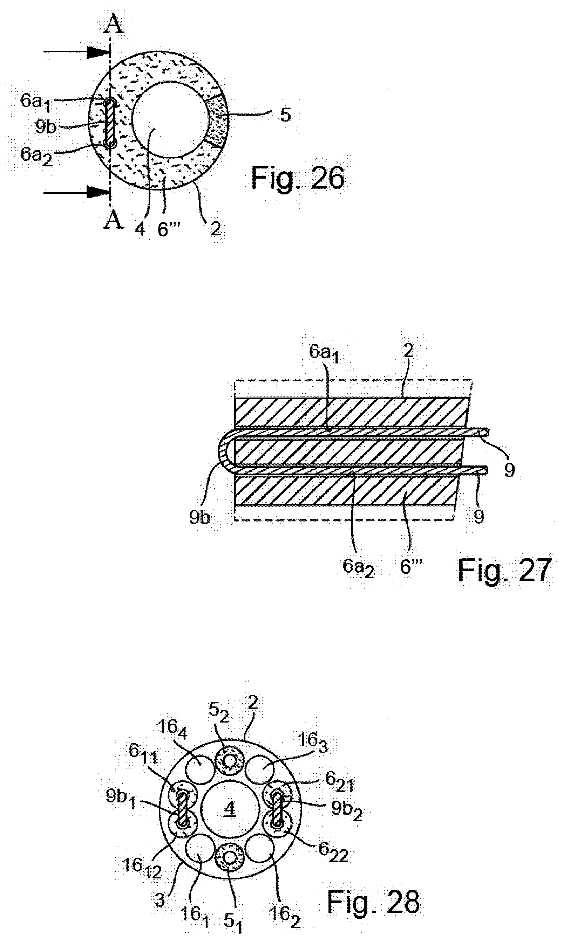

Die Fig. 26 bis 28 zeigen Ausführungsformen der Erfindung bei denen jeweils ein durch zwei Hohlkanäle durchgeschleifter Zugdraht als Biegesteuerelement fungiert, wodurch eine separate Fixierung eines distalen Zugdrahtendes am distalen Abschnitt entfallen kann. Die Fig. 26 und 27 veranschaulichen dazu ein Ausführungsbeispiel, das von der Kathetervariante der Fig. 24 und 25 ausgeht und demgegenüber dahingehend modifiziert ist, dass anstelle des einen Hohlkanals 6a zwei benachbarte Hohlkanäle 6a₁, 6a₂ in die Biegeelement-Vollmaterialzone 6'" des Vollmaterials des distalen Abschnitts 2 und korrespondierend in das Vollmaterial des Schaftabschnitts eingebracht sind. Ein einteiliger Zugdraht 9 erstreckt sich durch den einen Hohlkanal, z.B. den Hohlkanal 6a₁, bis zum distalen Ende des distalen Abschnitts 2 und ist von dort unter Bildung eines entsprechenden Umkehrbogens 9b durch den anderen Hohlkanal, z.B. den Hohlkanal 6a₂, bis zum proximalen Ende des Katheterschlauchs zurückgeführt.

Zum Biegen des distalen Abschnitts 2 wird an beiden proximalen Enden des durch die beiden benachbarten Hohlkanäle 6a₁, 6a₂ durchgeschleiften Zugdrahts 9 synchron gezogen. Der Umlenkbogen 9b des Zugdrahts 9 überträgt die Zugkraft auf die Biegeelement-Vollmaterialzone 6'" des distalen Abschnitts 2, wodurch dessen gewünschte Biegung bewirkt wird. Eine separate Zugdrahtfixierung am distalen Abschnitt 2 kann entfallen, und es entfällt auch die Notwendigkeit, ein distales Zugdrahtende in spezieller Weise zu gestalten, wie beispielsweise im Fall des Zugdraht-Kopfteils 9a beim Katheterinstrument gemäß den Fig. 4 bis 6.

Die Verwendung eines solchen, durch zwei benachbarte Hohlkanäle durchgeschleiften Zugdrahtes ist selbstverständlich auch für alle anderen Kathetervarianten möglich. Fig. 28 zeigt hierzu eine Kathetervariante, die vom Ausführungsbeispiel gemäß Fig. 12 ausgeht und als Modifikation die Maßnahme beinhaltet, dass das jeweilige Biegeelement statt des Biegeelementstabes 6₁, 6₂ nebst zugehörigem Zugdraht zwei benachbarte Biegeelementstäbe 6₁₁, 6₁₂ bzw. 6₂₁, 6₂₂ mit je einem Zugdrahtdurchführungs-Hohlkanal beinhaltet, wobei jeweils ein einteiliger Zugdraht 9₁, 9₂ durch die beiden Hohlkanäle der zwei benachbarten Biegeelementstäbe 6₁₁, 6₁₂ bzw. 6₂₁, 6₂₂ in der oben zu den Fig. 26 und 27 beschriebenen Weise durchgeschleift ist. Der Umlenkbogen 9b₁, 9b₂ jedes dieser beiden durchgeschleiften Zugdrähte 9₁, 9₂ bildet wiederum das betreffende Mittel zur Kraftübertragung vom jeweiligen durchgeschleiften Zugdraht 9₁, 9₂ auf die beiden zugehörigen Biegeelementstäbe 6₁₁, 6₁₂ bzw. 6₂₁, 6₂₂.

Die Verwendung eines bzw. mehrerer durchgeschleifter Zugdrähte, wie zu den Ausführungsbeispielen der Fig. 26 bis 28 erläutert, ermöglicht es, die Zugdrahtdurchführungsöffnungen kleiner als im Fall eines nur bis zum distalen Ende geführten und dort fixierten Zugdrahtes zu wählen, da sich die Zugkraft auf die beiden synchron betätigten Zugdrahtteile aufteilt. Der Umlenkbogen sorgt ohne Notwendigkeit einer weiteren Zugdrahtfixierung für eine formschlüssige Verbindung des Zugdrahts mit dem bzw. den zugehörigen Biegeelementen. Durch den Wegfall einer distalen Zugdrahtfixierung braucht auch nicht für eine ausreichende Sicherheit dieser Fixierung gesorgt zu werden.

Wie die obigen Erläuterungen vorteilhafter Ausführungsbeispiele deutlich machen, stellt die Erfindung ein medizinisches Katheterinstrument bereit, dessen distaler Abschnitt sich in einer sehr vorteilhaften Weise nach Bedarf aktiv und gesteuert biegen lässt bzw. in seiner Biegestellung einstellbar ist. Die Biegesteuerung wird vorzugsweise durch Zugdrähte von zu diesem Zweck an sich bekannter Art bewirkt, z.B. durch Zugdrähte aus einem Volldrahtmaterial, einem Litzenmaterial oder einem Kunstfasermaterial. Bei Verwendung von nichtmetallischen bzw. nichtmagnetischen Werkstoffen im distalen Abschnitt eignet sich das gesteuert biegbare Katheterinstrument unter anderem auch für den Einsatz bei Untersuchungen mit bildgebenden Magnetresonanzverfahren. Durch Dotierungen einzelner Komponenten des distalen Abschnitts mit röntgensichtbaren Materialien kann bei Bedarf die Röntgensichtbarkeit des Katheterinstruments verbessert werden. Bei Anwendungen mit bildgebender Magnetresonanz kann die Sichtbarkeit auch durch unter Magnetresonanz sichtbare Beschichtungen oder Dotierungen verbessert werden. Das erfindungsgemäße Katheterinstrument ist in einem distalen Abschnitt sowohl aktiv in seiner Biegung steuerbar als auch im passiven Zustand elastisch, was Gewebeverletzungen vorbeugt.

## Patentansprüche

1. Medizinisches Katheterinstrument mit
- einem Schaftabschnitt (1),
- einem gegenüber dem Schaftabschnitt in wenigstens einer Richtung gesteuert biegbaren distalen Abschnitt (2) und
- wenigstens einem sich axial beweglich durch den Schaftabschnitt hindurch zum distalen Abschnitt erstreckenden Steuerelement (9), dessen Axialbewegung eine korrespondierende Biegebewegung des distalen Abschnitts steuert,
- wenigstens einer Biegeanordnung mit wenigstens einem langgestreckten, elastisch biegbaren Versteifungselement (5) und wenigstens einem langgestreckten, biegbaren und axial längenveränderbaren Biegeelement (6"), die wenigstens in einem axialen Teilbereich des distalen Abschnitts vorgesehen sind und sich in diesem mit axialer Hauptkomponente erstrecken, wobei das Versteifungselement ein biegebewegungsrückstellendes Element mit höherer Biegesteifigkeit als das wenigstens eine Biegeelement bildet und das Biegeelement an das wenigstens eine Steuerelement gekoppelt ist oder einen Teil desselben bildet,
**dadurch gekennzeichnet, dass**
- das Steuerelement durch einen Zugdraht (9) gebildet ist, der mit einem distalen Endbereich des Biegeelements (6") gekoppelt ist, und das Biegeelement (6") zwei Hohlkanäle (6a₁, 6a₂) aufweist, durch die der Zugdraht einteilig unter Bildung eines Umkehrbogens (9b) am distalen Endbereich des Biegeelements und zweier synchron zugbetätigter proximaler Enden durchgeführt ist.

2. Katheterinstrument nach Anspruch 1, weiter **dadurch gekennzeichnet, dass** sich das Versteifungselement vom distalen Abschnitt in den Schaftabschnitt hinein fortsetzt.

3. Katheterinstrument nach Anspruch 1 oder 2, weiter **dadurch gekennzeichnet, dass** das Biegeelement durch ein elastisch druckbelastbares Schraubenfeder- oder Hohlstabelement (6, 6') oder eine elastisch druckbelastbare Vollmaterialzone (6") des distalen Abschnitts gebildet ist.

4. Katheterinstrument nach einem der Ansprüche 1 bis 3, weiter **dadurch gekennzeichnet, dass** das Versteifungselement durch ein elastisch zugbelastbares Schraubenfeder- oder Stabelement (5, 5', 5") oder eine elastisch zugbelastbare Vollmaterialzone (5"') des distalen Abschnitts gebildet ist.

5. Katheterinstrument nach einem der Ansprüche 1 bis 4, weiter **dadurch gekennzeichnet, dass** das Versteifungselement und das Biegeelement einander bezüglich einer Längsmittenachse des distalen Abschnitts gegenüberliegend angeordnet sind.

6. Katheterinstrument nach einem der Ansprüche 1 bis 4, weiter **dadurch gekennzeichnet, dass** die Biegeanordnung wenigstens zwei Biegeelemente (6₁ 6₂), die einander in einer Ebene der Biegebewegung gegenüberliegend angeordnet sind, und wenigstens zwei Versteifungselemente (5₁, 5₂) umfasst, die nebeneinander in einer Ebene quer zur Ebene der Biegebewegung angeordnet sind.

7. Katheterinstrument nach einem der Ansprüche 1 bis 6, weiter **dadurch gekennzeichnet, dass** der distale Abschnitt aus einem Vollmaterial (3) oder aus zwei koaxialen Schlauchelementen (17, 18) aufgebaut ist, wobei die Biegeanordnung im Vollmaterial oder in einem Ringraum (20) zwischen den koaxialen Schlauchelementen vorgesehen ist.

8. Katheterinstrument nach einem der Ansprüche 1 bis 7, weiter **dadurch gekennzeichnet, dass** der distale Abschnitt einen oder mehrere außermittige axiale Nutzkanäle (5a, 5₁a, 5₂a, 16₁, 16₂, 16₃, 16₄) aufweist.

9. Katheterinstrument nach Anspruch 8, weiter **dadurch gekennzeichnet, dass** der distale Abschnitt aus einem den mittigen axialen Nutzkanal (4') mit ungleichförmiger Dicke umgebenden Vollmaterial aufgebaut ist, das in einem Bereich geringerer Dicke eine elastisch zugbelastbare Vollmaterialzone (5₆) als Versteifungselement beinhaltet.

10. Katheterinstrument nach Anspruch 9, weiter **dadurch gekennzeichnet, dass** das Vollmaterial eine elastisch druckbelastbare Zone (6"'), die einen Bereich größerer Dicke des Vollmaterials umfasst, als Biegeelement beinhaltet.

11. Katheterinstrument nach einem der Ansprüche 1 bis 10, weiter **dadurch gekennzeichnet, dass** mehrere Biegeanordnungen, die jeweils wenigstens ein Versteifungselement und wenigstens ein Biegeelement aufweisen, in verschiedenen axialen Teilbereichen (2₁, 2₂) des distalen Abschnitts vorgesehen sind, wobei eine erste und eine zweite Biegeanordnung zueinander nichtparallele Biegebewegungsebenen aufweisen.

12. Katheterinstrument nach einem der Ansprüche 1 bis 11, weiter **dadurch gekennzeichnet, dass** der distale Abschnitt wenigstens ein Lichtleiterelement (22₁, 22₂) und einen an dieses angekoppelten Leuchtring (23) aufweist, wobei das Lichtleiterelement axial verlaufend in einem Nutzkanal des distalen Abschnitts aufgenommen ist.

13. Katheterinstrument nach einem der Ansprüche 1 bis 12, weiter **dadurch gekennzeichnet, dass** der distale Abschnitt ein axial elastisch bewegliches distales Endstück (25) aufweist, das mit wenigstens einem Führungsstift (26₁, 26₂) verbunden ist, der in einem axialen Hohlkanal (24₁, 24₂) des distalen Abschnitts axial elastisch beweglich geführt ist.

14. Katheterinstrument nach einem der Ansprüche 1 bis 13, weiter **dadurch gekennzeichnet, dass** eine oder mehrere Komponenten des distalen Abschnitts eine Beschichtung mit einem röntgensichtbaren Material oder eine unter Magnetresonanz sichtbare Beschichtung oder Dotierung aufweisen.

## Claims

1. Medical catheter instrument, comprising
- a shaft portion (1),
- a distal portion (2) bendable in a controlled manner in at least one direction relative to the shaft portion, and
- at least one control element (9) extending in an axially movable manner through the shaft portion to the distal portion, the axial movement of the control element controlling a corresponding bending movement of the distal portion,
- at least one bending assembly comprising at least one elongated, elastically bendable stiffening element (5) and at least one elongated, bendable and axially length-variable bending element (6"), which are provided at least in an axial subregion of the distal portion and extend therein with an axial main component, wherein the stiffening element forms a bending movement restoring element which has a higher flexural rigidity than the at least one bending element, and the bending element is coupled to the at least one control element or forms a part of the control element,
**characterized in that**
- the control element is formed by a tension wire (9) which is coupled to a distal end region of the bending element (6"), and the bending element (6") has two hollow channels (6a₁, 6a₂), through which the tension wire is guided in one piece so as to form a return bend (9b) at the distal end region of the bending element and two proximal ends tensionally actuated in synchronization.

2. Catheter instrument according to claim 1, further **characterized in that** the stiffening element extends from the distal portion into the shaft portion.

3. Catheter instrument according to claim 1 or 2, further **characterized in that** the bending element is formed by an elastically compressible helical spring or hollow rod element (6, 6'), or an elastically compressible solid material zone (6") of the distal portion.

4. Catheter instrument according to any one of claims 1 to 3, further **characterized in that** the stiffening element is formed by an elastically tensible helical spring element or rod element (5, 5', 5"), or an elastically tensible solid material zone (5"') of the distal portion.

5. Catheter instrument according to any one of claims 1 to 4, further **characterized in that** the stiffening element and the bending element are arranged opposite to each other relative to a longitudinal central axis of the distal portion.

6. Catheter instrument according to any one of claims 1 to 4, further **characterized in that** the bending assembly comprises at least two bending elements (6₁, 6₂) which are arranged opposite to each other in a plane of the bending movement, and at least two stiffening elements (5₁, 5₂) which are arranged side by side in a plane transversely to the plane of the bending movement.

7. Catheter instrument according to any one of claims 1 to 6, further **characterized in that** the distal portion is made of solid material (3) or composed of two coaxial tubular elements (17, 18), wherein the bending assembly is provided in the solid material or in an annular space (20) between the coaxial tubular elements.

8. Catheter instrument according to any one of claims 1 to 7, further **characterized in that** the distal portion has one or more off-centered axial working channels (5a, 5₁a, 5₂a, 16₁, 16₂, 16₃, 16₄).

9. Catheter instrument according to claim 8, further **characterized in that** the distal portion is composed of a solid material surrounding the central axial working channel (4') in non-uniform thickness, which solid material includes, in a region of lesser thickness, an elastically tensible solid material zone (5₆) as a stiffening element.

10. Catheter instrument according to claim 9, further **characterized in that** the solid material includes an elastically compressible zone (6"'), which comprises a region of greater thickness of the solid material as a bending element.

11. Catheter instrument according to any one of claims 1 to 10, further **characterized in that** a plurality of bending assemblies, each of which has at least one stiffening element and at least one bending element, are provided in different axial subregions (2₁, 2₂) of the distal portion, wherein a first and a second bending assembly have bending movement planes that extend non-parallel to each other.

12. Catheter instrument according to any one of claims 1 to 11, further **characterized in that** the distal portion includes at least one light guide element (22₁, 22₂) and, coupled thereto, a luminous ring (23), wherein the light guide element is accommodated in an axially extending manner within a working channel of the distal portion.

13. Catheter instrument according to any one of claims 1 to 12, further **characterized in that** the distal portion comprises a distal end piece (25) moveable in an axially elastic manner which is connected to at least one guide pin (26₁, 26₂), which pin is guided within an axial hollow channel (24₁, 24₂) of the distal portion to be moved in an axially elastic manner.

14. Catheter instrument according to any one of claims 1 to 13, further **characterized in that** one or more components of the distal portion include a coating comprising an X-ray visible material, or a coating or doping visible with magnetic resonance.

## Revendications

1. Instrument médical à cathéter, avec
- une partie de tige (1),
- une partie distale (2) pouvant être courbée de façon ciblée dans au moins une direction par rapport à la partie de tige, et
- au moins un élément de commande (9) s'étendant de façon mobile axialement à travers la partie de tige vers la partie distale, et dont le mouvement axial commande un mouvement de courbure correspondant de la partie distale,
- au moins un agencement de courbure avec au moins un élément de raidissement allongé (5) pouvant être courbé élastiquement et au moins un élément de courbure allongé (6") pouvant être courbé et de longueur axialement variable, qui sont prévus au moins dans une région partielle axiale de la partie distale et qui s'étendent dans celle-ci avec une composante principale axiale, dans lequel l'élément de raidissement forme un élément de rappel du mouvement de courbure avec une rigidité de courbure plus élevée que ledit au moins un élément de courbure et l'élément de courbure est couplé audit au moins un élément de commande ou forme une partie de celui-ci,
**caractérisé en ce que**
- l'élément de commande est formé par un fil de traction (9), qui est couplé avec une région d'extrémité distale de l'élément de courbure (6"), et l'élément de courbure (6") présente deux canaux creux (6a₁, 6a₂), à travers lesquels le fil de traction est mené d'une seule pièce avec formation d'un arc d'inversion (9b) à la région d'extrémité distale de l'élément de courbure et de deux extrémités proximales pouvant être actionnées par traction de façon synchrone.

2. Instrument médical à cathéter selon la revendication 1, **caractérisé en outre en ce que** l'élément de raidissement se prolonge depuis la partie distale jusque dans la partie de tige.

3. Instrument médical à cathéter selon la revendication 1 ou 2, **caractérisé en outre en ce que** l'élément de courbure est formé par un élément de ressort hélicoïdal ou de barre creuse (6, 6') pouvant être soumis à la compression élastiquement ou par une zone de matière massive (6") pouvant être soumise à la compression élastiquement de la partie distale.

4. Instrument médical à cathéter selon l'une quelconque des revendications 1 à 3, **caractérisé en outre en ce que** l'élément de raidissement est formé par un élément de ressort hélicoïdal ou de barre (5, 5', 5") pouvant être soumis à la traction élastiquement ou par une zone de matière massive (5"') pouvant être soumise à la traction élastiquement de la partie distale.

5. Instrument médical à cathéter selon l'une quelconque des revendications 1 à 4, **caractérisé en outre en ce que** l'élément de raidissement et l'élément de courbure sont disposés l'un en face de l'autre par rapport à un axe central longitudinal de la partie distale.

6. Instrument médical à cathéter selon l'une quelconque des revendications 1 à 4, **caractérisé en outre en ce que** l'agencement de courbure comprend au moins deux éléments de courbure (6₁, 6₂), qui sont disposés l'un en face de l'autre dans un plan du mouvement de courbure, et comprend au moins deux éléments de raidissement (5₁, 5₂), qui sont disposés l'un à côté de l'autre dans un plan transversal au plan du mouvement de courbure.

7. Instrument médical à cathéter selon l'une quelconque des revendications 1 à 6, **caractérisé en outre en ce que** la partie distale est constituée d'une matière massive (3) ou de deux éléments de tuyau coaxiaux (17, 18), dans lequel l'agencement de courbure est prévu dans la matière massive ou dans une chambre annulaire (20) entre les éléments de tuyau coaxiaux.

8. Instrument médical à cathéter selon l'une quelconque des revendications 1 à 7, **caractérisé en outre en ce que** la partie distale présente un ou plusieurs canal/canaux utilitaire(s) axial/axiaux décentré(s) (5a, 5₁a, 5₂a, 16₁, 16₂, 16₃, 16₄).

9. Instrument médical à cathéter selon la revendication 8, **caractérisé en outre en ce que** la partie distale est constituée d'une matière massive entourant le canal utilitaire axial central (4') avec une épaisseur irrégulière, et qui contient dans une région de plus faible épaisseur une zone de matière massive (5₆) pouvant être soumise à la traction élastiquement en tant qu'élément de raidissement.

10. Instrument médical à cathéter selon la revendication 9, **caractérisé en outre en ce que** la matière massive contient en tant qu'élément de courbure une zone (6'") pouvant être soumise à la compression élastiquement, qui comprend une région de plus forte épaisseur de la matière massive.

11. Instrument médical à cathéter selon l'une quelconque des revendications 1 à 10, **caractérisé en outre en ce qu'**il est prévu plusieurs agencements de courbure, qui présentent respectivement au moins un élément de raidissement et au moins un élément de courbure, dans des régions partielles axiales différentes (2₁, 2₂) de la partie distale, dans lequel un premier et un deuxième agencements de courbure présentent des plans du mouvement de courbure non parallèles l'un à l'autre.

12. Instrument médical à cathéter selon l'une quelconque des revendications 1 à 11, **caractérisé en outre en ce que** la partie distale présente au moins un élément de guide de lumière (22₁, 22₂) et un anneau lumineux (23) couplé à celui-ci, dans lequel l'élément de guide de lumière s'étendant axialement est contenu dans un canal utilitaire de la partie distale.

13. Instrument médical à cathéter selon l'une quelconque des revendications 1 à 12, **caractérisé en outre en ce que** la partie distale présente une pièce d'extrémité distale (25) mobile axialement de façon élastique, qui est reliée à au moins une tige de guidage (26₁, 26₂), qui est guidée en mouvement axial élastique dans un canal creux axial (24₁, 24₂) de la partie distale.

14. Instrument médical à cathéter selon l'une quelconque des revendications 1 à 13, **caractérisé en outre en ce qu'**un ou plusieurs composant(s) de la partie distale présente(nt) un revêtement avec un matériau visible aux rayons X ou un revêtement ou un dopage visible par résonance magnétique.
